(19) Europäisches Patentamt
European Patent Office
Office européen des brevets

(11) Publication number: 0 335 477 B1

(12) # EUROPEAN PATENT SPECIFICATION

(45) Date of publication of patent specification: **21.07.93** (51) Int. Cl.$^5$: **A61K 7/13**

(21) Application number: **89300514.0**

(22) Date of filing: **19.01.89**

The file contains technical information submitted after the application was filed and not included in this specification

(54) **The use of N-substituted-5,6-dihydroxyindoles as a hair coloring agent.**

(30) Priority: **31.03.88 US 175565**

(43) Date of publication of application: **04.10.89 Bulletin 89/40**

(45) Publication of the grant of the patent: **21.07.93 Bulletin 93/29**

(84) Designated Contracting States: **AT BE CH DE ES FR GB GR IT LI LU NL SE**

(56) References cited:
**GB-A- 2 132 642**
**GB-A- 2 187 456**

(73) Proprietor: **Bristol-Myers Company**
**345 Park Avenue**
**New York New York 10154(US)**

(72) Inventor: **Schultz, Thomas M.**
**25, Dogwood Court**
**Highland Mills, N.Y. 10930(US)**
Inventor: **Brown, Keith C.**
**59, Douglas Road**
**New Canaan Connecticut 06840(US)**
Inventor: **Murphy, Bryan P.**
**94, Hillside Lane**
**Monroe Connecticut 06468(US)**
Inventor: **Mayer, Alice A.**
**37, Juniper Road**
**Bethel Connecticut 06801(US)**
Inventor: **Lim,, Mu-Ill**
**31, Mayflower Drive**
**Trumbull Connecticut 06611(US)**

(74) Representative: **Baillie, Iain Cameron et al**
**Ladas & Parry Altheimer Eck 2**
**W-8000 München 2 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.6/3.3.1)

## Description

This invention relates to a method for dyeing hair using N-substituted-5, 6-dihydroxyindoles as a hair coloring agent.

### Background of the Invention

Over the past thirty years the prior art has disclosed the use of dihydroxyindoles as hair dyes. The major thrust of the teachings of the prior art is that 5,6-dihydroxyindole (DHI) is a metabolite of DOPA and is intimately related to the natural mammalian pigment eumelanin. Various prior art references teach that a natural looking dyeout can be obtained using solutions of 5,6-dihydroxyindole. It is known, however, that use of this dye agent alone requires long dyeout times and therefore a variety of accelerators has been employed to hasten the dye formation.

It is also known that DHI is difficult to synthesize requiring exceptional care in its preparation. The compound is unstable in air and rapidly decomposes to other materials which are ineffective as hair colorants. Therefore, commercial production of DHI has been lacking and this may be the major reason why no hair coloring products utilize dihydroxyindoles.

In the prior art that teaches the use of dihydroxyindoles as hair colorants, commonly the dye solution is of such a nature that the oxidants used as accelerators: must be kept separate from the dihydroxyindoles, and the two parts are typically mixed together immediately before use. However, dyeing methods are described wherein the dye/oxidant combination can be applied sequentially.

Specifically, U.S. patent No. 2,934,396 teaches that DHI in an acidic solution should be applied to the hair followed by a second application of an amine (alkaline) base or an amine base with $IO_3$, $IO_4$, persulfate, $CuSO_4$ or $MnSO_4$ to initiate the dyeout. The range of brown shades is claimed but dyeing times are long and from about 45 minutes for light shades to about 90 minutes for the darker browns. The desirability of keeping the DHI solution at acidic pH is a result of the inherent instability of the compound. One method of overcoming this difficulty in the handling of DHI has been taught in U.S. patent No. 3,194,734 where the more stable diacetate of DHI is converted to the free phenol with an alkalizing agent immediately prior to use. Otherwise the alkaline DHI solution will spontaneously form particulate matter and become ineffective for dyeing hair.

G.B. 887,579 and U.S. patent No. 3,194,734 both teach that acidic to alkaline solutions of the 1-, 2-, or 3-methyl substituted dihydroxyindoles or combinations thereof color hair to a variety of brown shades depending upon the composition mixture. In these cases the process involves applying first either an alkaline or acidic mixture of the methyl-5,6-dihydroxyindole to hair followed by contacting the hair with an oxidizing solution. Alternatively, immediately prior to use, the acidic indole solution is mixed with the alkaline oxidizing solution and then this mixture is applied to the hair. These patents claim that various light to dark brown dyeouts are obtained over about 40 to about 60 minutes.

Whereas the prior art teaches that DHI or its methylated derivatives dye hair dark brown to black in 60 to 90 minutes, the resulting color can be somewhat modulated by incorporating a metal-ion into the dye composition. Specifically it is taught in US 3,194,734 that applying a neutral to basic pH solution of the 5,6-dihydroxyindole and an oxidizing solution containing a metal salt, such as cobaltous acetate or silver nitrate will color the hair light brown to reddish brown. It is specifically taught that the solutions are to be made up fresh and then used immediately. Even with metal catalyst added to the dye mixture the dyeing time is still long.

A significant improvement in the prior art is taught in Dutch Patent N1 8304157A wherein hair is treated with a metal salt solution (salts of Co, Fe, Mn, Ti, Cu, ZN, Ta, Cr, Ni, Pd, Pt, Au, Mg, Cd, Br, Sn, Pb, or Bi) prior to application of an alkaline solution of DHI. Color formation in hair requires in this case only a few minutes. However, it should be noted that, although highly intense, the initial color imparted is achromatic.

Application GB-A-2,187,4555 (L'Oreal) discloses a process for dyeing hair comprising contacting the hair with a solution of water soluble metal followed by contacting the hair with a solution of a N-(lower alkyl) substituted-5-6 dihydroxyindole derivative.

We have now found that N-substituted dihydroxyindoles dye metal ion-pretreated hair to a wide range of cosmetically pleasing colors. The imparted colors are controlled by the nature of the metal ion, its concentration and pH of the dyeing composition.

## Summary of the Invention

The present invention refers to a method for dyeing hair which comprises contacting the hair with a solution of a water soluble metal ion followed by contacting the hair with a solution of a N-substituted-5,6-dihydroxyindole, or in the reverse order.

Particular embodiments of the invention are described in the dependent claims.

## Detailed Description of the Invention

The art of hair dyeing with melanin precursors such as DHI has been severely hindered by the instability of the dye. We were surprised to find that pH 3-12 solutions of N-substituted-5, 6-dihydroxyindoles were exceptionally stable and could be handled in the open air with no extraordinary precautions taken.

Furthermore, although their oxidation is slow, metal ion-pretreated hair is dyed a wide range of colors by N-substituted-5, 6-dihydroxyindoles within a few minutes.

This variety of colors is achieved simply by use of different metal salts. The color intensity can be further modulated by the concentration of the chosen metal salts and/or the pH of the color-forming reactions.

Preferably, it is the identity of the metal ion which is varied to change the color. For example, use of Cu(II) yields a black whereas Zn (II) gives a golden brown and Ag (I) yields a dark auburn. In the absence of metal ions the dyeing process is slow and the color product is a drab brown. But in the presence of a metal-ion a dramatic acceleration in the speed of the dyeing occurs and the total process is over in a few minutes.

Because the dyeing process does not require the use of the harsh oxidation chemicals frequently incorporated in commercial permanent hair coloring products, hair is not oxidatively damaged and feels full and smooth. Moreover both the metal salts and dihydroxyindoles may be applied from conditioning shampoos further contributing to a pleasant feel of the hair.

Although the hair can be either treated first with the indole dye mixture followed by the metal salt or in the reverse order, the results obtained by a metal-ion pretreatment are generally superior.

In addition, the imparted color can be further modulated or substantially removed by treatment of the dyed hair with oxidants such as $H_2O_2$ or reductants such as sulfites, etc.

Another significant advantage to the use of N-substituted-5,6-dihydroxyindole is their significantly easier synthesis. Whereas the synthesis route to DHI requires many steps, some of which involve extremely harsh chemical reagents, the N-substitutued materials are readily synthesized from the corresponding 3,4-dihydroxy-phenethylamine precursors, and quite unexpectedly, the N-substituted-5, 6-dihydroxyindoles so produced are extremely stable.

Consequently, they do not require storage under nitrogen or in poly-alcohols, as has been taught for DHI itself.

By way of specific examples, we show the preparation of derivatives of N-substituted-5,6-dihydroxyindoles (I) which demonstrates the ease of their preparation.

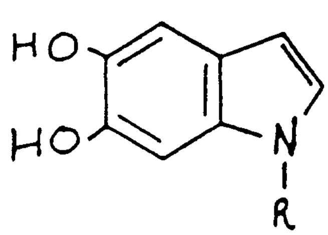

I

The following is intended to illustrate without limitation thereto various known routes of synthesis of various N-substituted-5,6-dihydroxyindoles useful in the present invention.

(a) Synthesis by cyclization of a Dopamine derivative:

The suitable 3,4-dihydroxy-N-substitutued phenethylamine material is converted by oxidative cyclization followed by chemical reduction to the N-substituted-dihydroxyindole as per the method for preparation of N-methyl-5,6-dihydroxyindoles from epinephrine described by G.L. Mattok and R. A. Heacock in the Canadian Journal of Chemistry, Volume 42, p.484 (1964), but the invention is not to be limited by the

exact use of solvent and oxidants detailed therein.

For example, N-isopropyl-3,4-dihydroxyphenethylamine (II) can be oxidized by an alkaline ferricyanide solution followed by reduction with ascorbic acid to give N-isopropyl-5,6-dihydroxyindole (III):

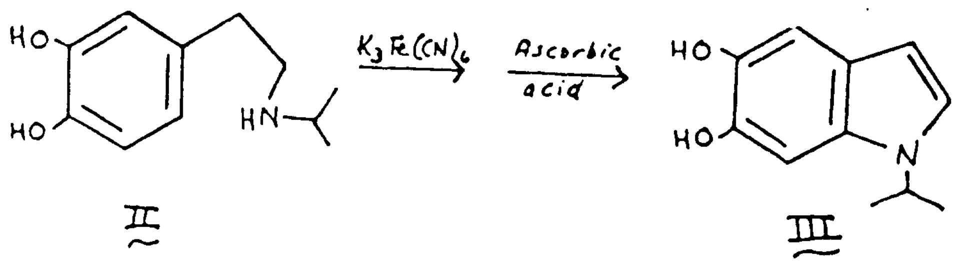


II

III

Similarly N-(4-aminobutyl)-5-6-dihydroxyindole (V) can be obtained from 3,4-dihydroxy-N-(4-nitrobutyl)-phenethylamine (IV);

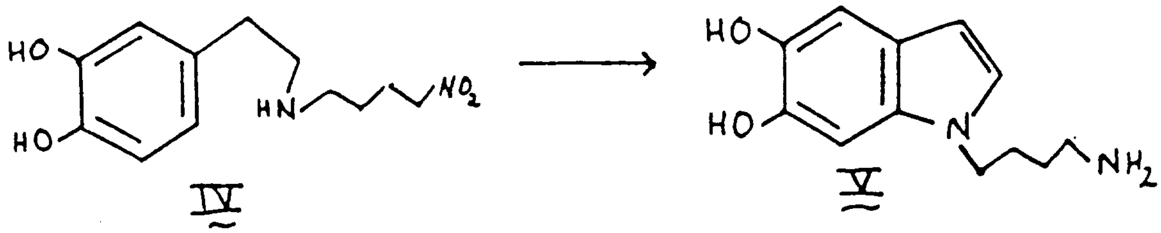

IV

V

(b) Synthesis by condensation of a benzenoid compound and a nitrogen containing compound.

An alternate synthetic procedure is available by the method described by A. Reissert in Chemische Berichte, Volume 30, page 1030 (1897) and C.D. Nenitzecu in Bulletin of the Chemistry Society of Rumania, Volume 11, page 37 (1929) where the indole i produced upon the condensation of a benzenoid radical with an appropriate N-containing material. For example, N-(2-hydroxy-ethyl)5,6-dihydroxyindole (VI) can be obtained upon oxidative coupling of catechol (VII) with N-vinyl ethanolamine (VIII followed by reduction.

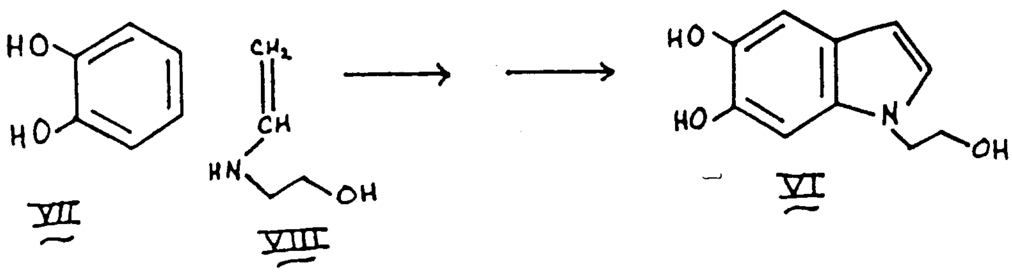

VII

VIII

VI

(c) Synthesis by addition of indole to an aryl compound A final example is the direct addition of the indole to an aryl or benzyl halide or azide to give the aryl or arylalkyl N-substituted-5,6-dihydroxyindole. Specifically, 5,6-diacetoxy-indole (IX) is reacted with fluoro-2,4-dinitrobenzene (X) to give after hydrolysis of the acetate radicals, N-(2,4-dinitrophenyl)5, -6-dihydroxyindole (XI);

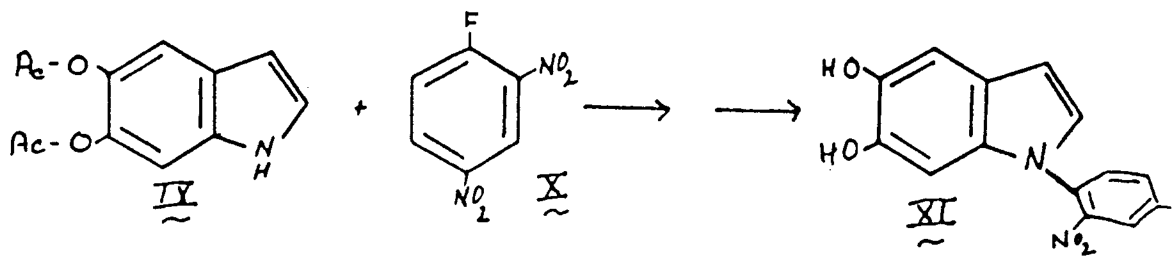

The N-subsituents according to present the invention will be those derived from structure I where the N-substituent, R, is alkyl, alkoxy, hydroxyalkyl, aminoalkyl, aminoaryl, and nitroaryl, wherein the aryl group may bear from 1 to 3 substituents.

The preferred indoles are those in which R is $C_1$-$C_8$ alkyl, $C_1$-$C_8$ alkoxy, $C_1$-$C_8$ hydroxyalkyl, or $C_1$-$C_8$ aminoalkyl. Substituents on the aryl groups may be nitro, alkyl, amino, alkoxy, or hydroxy and dinitro, diamino, or dihydroxyl.

## EXAMPLES

Alkaline solutions of metal salts were prepared in amine (for example, monoethanolamine) buffers while those at lower pH were adjusted with citric acid.

N-substituted indole were dissolved in aqueous alcohols (ethanol, propanol etc.) The pH of these solutions were adjusted either with ethanolamine or citric acid.

The following examples are listed to demonstrate the scope of this invention. Examples II through IX illustrate the color modulation of dyeouts from N-methyl-5, 6-dihydroxyindole attained on metal-ion pretreated hair.

### Example I:

Blended grey hair was treated with a 1% solution of N-methyl-5,6-dihydroxyindole at pH 8.5 for 50 minutes. A light brown color was produced.

### Example II:

Blended grey hair was treated with a pH 9 solution of 1.0% $CuSO_4$ for 5 minutes at ambient temperature, rinsed, and then exposed to a pH solution of N-methyl-5,6-dihydroxyindole (1% wt/wt for 5 minutes. The hair was dyed very dark grey with yellow tones.

### Example III:

The method of Example II was repeated, but the N-methyl-5, 6-dihydroxyindole solution was at pH 3 resulting in a dark charcoal grey dyeout.

### Example IV:

The method of Example II was repeated, but the solution of N-methyl-5, 6-dihydroxyindole solution was at pH 12. The hair color was black.

### Example V:

Blended grey hair was treated with 0.1% $Fe_2(SO_4)_3$ solution at pH 6 for 5 minutes rinsed and then soaked in a pH 8.5 solution of 1% (wt/wt) N-methyl-5,6-dihydroxyindole for 5 minutes to give a grey-brown color.

**Example VI:**

Blended grey hair was treated with a pH 5.5 0.1% solution of $Mn(OAc)_2$ for 5 minutes, rinsed, and then soaked in a 1% (wt/wt) solution of N-methyl-5,6-dihydroxyindole for 5 minutes to give a light grey-brown dyeout.

**Example VII:**

Blended grey hair was treated with 0.1% $AgNO_3$ at pH 5 for 5 minutes and then in a 1% N-methyl-5,6-dihydroxyindole solution at pH 6 for 5 minutes. The hair was dyed dark auburn-brown.

**Example VIII:**

The method of Example VII was repeated, using a 0.001% $AgNO_3$ solution which yielded a silvery light brown.

**Example IX:**

Blended grey hair was treated with a 0.1% (wt/wt) in Zn $(OAc)_2$ solution at pH 6 for 5 minutes, rinsed, and then with a 1% (wt/wt) N-methyl-5,6-dihydroxyindole solution at pH 9 to give a golden brown dyeout in 5 minutes.

Examples X through XIII are given to demonstrate the narrower range of colors attainable upon pretreatment with the indole and post-treating with the metal salt solution.

**Example X:**

Blended grey hair was soaked in a pH 6 solution of N-methyl-5,6-dihydroxyindole (1% wt/wt) for 15 minutes, rinsed, and then treated for 5 minutes in a pH 8 solution of 1.0% $CuSO_4$ to give a dark grey-brown dyeout with yellow hues.

**Example XI:**

The method of Example V was repeated, but the order of soakings was reversed to the indole first, then in a pH 6 0.1% $Fe_2(SO_4)_3$ solution for 5 minutes to yield a light brown dyeout.

**Example XII:**

The method of Example VI was repeated but the order of treatments was reversed to give a dark brown dyeout.

**Example XIII:**

The method of Example VII was repeated but the order of treatment was reversed to give a dark auburn dyeout.

**Example XIV through XVI** detail the use of other N-substituted-5, 6-dihydroxyindoles found effective in coloring the hair durably.

**Example XIV:**

Blended grey hair was treated with a pH 9 solution of 1.0% $CuSO_4$ for 5 minutes, rinsed, and then exposed to a solution of 0.8% N-isopropyl-5,6-dihydroxyindole of pH 10 for 10 minutes to give a light brown color.

**Example XV:**

The method of Example XIV was repeated but the hair was first treated with 0.01% $AgNO_3$ at pH 8 for 5 minutes, rinsed, and then soaked in the 0.8% indole solution to give a light reddish brown color.

**Example XVI:**

Copper (II) pre-treated blended grey hair was immersed in a solution containing N-(2,4-dinitrophenyl)-5,6-dihydroxyindole (1% wt/wt at pH 9) for 15 minutes. The hair was dyed light brown with yellow tones.
Examples XVII through XXIV show the bleaching ability of both hydrogen peroxide and either bisulfite or thioglycollate in modulating or completely removing the color from N-substituted-5, 6-dihydroxyindole dyed blended grey hair.

| Example | Indole Structure I, R = | Metal-ion | Bleaching Agent | Color Before | Color After |
|---|---|---|---|---|---|
| XVII | R = $CH_3$ | none | 3% pH 9 $H_2O_2$ | light brown | golden brown |
| XVIII | R = $CH_3$ | $Cu^{+2}$ pretreatment | 3% pH 9 $H_2O_2$ | dark grey | light golden brown |
| XIX | R = $CH_3$ | $Fe^{+3}$ pretreatment | 3% pH 9 $H_2O_2$ | grey-brown | very light golden brown |
| XX | R = $CH_3$ | $Mn^{+2}$ pretreatment | 3% pH 9 $H_2O_2$ | light grey brown | light strawberry blonde |
| XXI | R = $CH_3$ | $Ag^{+1}$ pretreatment | 3% pH 9 $H_2O_2$ 10 minutes | dark auburn | medium reddish brown |
| XXII | R = $CH_3$ | $Zn^{+2}$ pretreatment | 3% pH 9 $H_2O_2$ pretreatment | golden brown | pale golden brown |
| XXIII | R = isopropyl | $Cu^{+2}$ pretreatment | 3% pH 9 $H_2O_2$ | light brown | pale grey |
| XXIV | R = $CH_3$ | $Cu^{+2}$ treatment | 3% pH 9 $H_2O_2$ 10 minutes | grey brown | medium grey |
| XXV | R = $CH_3$ | $Cu^{+2}$ pretreatment | 2% Bisulfite 10 minutes | dark grey | medium golden brown |
| XXVI | R = $CH_3$ | $Cu^{+2}$ pretreatment | 2% Thioglycollic acid 10 minutes | dark grey | light brown |

It has been found that optimum results can be obtained when the N-substituted-5,6-dihydroxyindoles of the present invention are utilized in an aerobic aqueous solution or in an alcoholic aqueous solution at

concentrations of from 0.1% to 2.0% (wt/wt). Above this limiting concentration no more intensity in the color of the dyeout is observed. Preferably the solution utilized will have a concentration of from 0.5% to 1.0% (wt/wt), and most preferably it will have a concentration of 1.0% (wt/wt).

Further, it has now been determined that the solution of N-substituted-5,6-dihydroxyindole will achieve maximum effective coloration of the hair to be dyed when said solution is applied at a pH of from pH 3 to pH 12, preferably pH 6 to pH 11, most preferably pH 7 to 10.

The utilization of the aforementioned optimal solution of N-substituted-5,6-dihydroxyindole together with a pre-treatment of the hair with a composition comprising water soluble salts of, $Ag^{+1}$, $Au^{+1}$, $Pb^{+2}$, $Pd^{+2}$, $Co^{+2}$, $Cu^{+2}$, $Bi^{+2}$, $Ni^{+2}$, $Mn^{+2}$, $Fe^{+3}$, $Cr^{+4}$ or $Ti^{+2}$ in concentration of from 0.0001 to 2% (wt/wt) and at a pH of from pH 3 to pH 11 has been found to be most effective in achieving a variety of desirable colorations in the dyed hair. Conventional water soluble counterions, e.g. sulfates, and nitrates, are useful.

The dyed hair may be subsequently treated with a solution of $H_2O_2$ in the concentration of from 1% to 6% (wt/wt), the preferred range is 1% to 3% (wt/wt), and at pH 6 to pH 10, with the preferred range of pH 8 to pH 10, for a period of 5 to 30 minutes to effect a rapid lightening of the hair to a light golden to light reddish brown coloration, as exemplified herein above.

## Claims

1. A method for dyeing hair which comprises contacting the hair with a solution of a water soluble metal ion followed by contacting the hair with a solution of a N-substituted-5,6-dihydroxyindole, or in the reverse order, wherein the substituent on the nitrogen atom is selected from the group consisting of: alkyl, alkoxy, hydroxyalkyl, aminoalkyl, aminoaryl, and nitroaryl, wherein the aryl group may bear from 1 to 3 substituents.

2. The method of claim 1 wherein said solution of N-substituted-5,6-dihydroxyindole is a 0.1 to 2.0 wt. % solution and wherein said solution of water soluble metal ion is a 0.001 to 2.0 wt. % solution.

3. The method of any preceding claim wherein the water soluble salts are selected from the group consisting of salts of Cr, Ag, Au, Pb, Pd, Co, Cu, Ni, Bi Mn, Fe, Ti, Sn, or Zn, and are applied in solution in concentraions of from 0.001% to 2.0% by weight at a pH of pH 3 to pH 11, prior to treating the hair with the said solution of N-substituted 5,6-dihydroxyindole.

4. The method of any preceding claim wherein the solution of the N-substituted-5,6-dihydroxyindole is applied at pH 3 to pH 12.

5. The method of claim 3 wherein the hair to be dyed is subjected to steps comprising: (1) treatment with a composition comprising water soluble salts of Cr, Ag, Au, Pb, Pd, Co, Cu, Ni, Bi, Mn, Fe, Ti, Sn, or Zn, applied in solution in concentrations of from 0.001% to 2% by weight at a pH of pH 3 to pH 11, (2) subsequent treatment with said soluion of N-substituted 5,6-dihydroxyindole at pH 3 to pH 12, either with or without rinsing of the first applied solution.

6. The method of claim 5 wherein the order of steps (1) and (2) is reversed.

7. The method of any preceding claim followed by the subsequent treatment of the hair with a solution of a bleaching agent, at a concentration of from 1% to 6% at from pH 6 to pH 11, for a period of 5 to 30 minutes to effect a lightening or removal of the coloration of the hair.

8. The method of claim 7 wherein the bleaching agent is $H_2O_2$.

9. The method of claim 7 wherein the bleaching agent is a salt of $SO_2$ such as sodium or potassium bisulfate or a sulfhydryl compound such as thioglycollic acid or its potassium, sodium or ammonium salts.

10. The method of any preceding claim wherein the substituent in the N-substituted-5,6-dihydroxyindole is methyl, isopropyl, an aminophenyl, or a nitrophynyl wherein each benzene ring may also have from 1 to 3 substituents.

11. The method of any preceding claim wherein the N-substituted-5,6-di-hydroxyindole is N-methyl-5,6-dihydroxyindole.

12. The method of any preceding claim wherein the N-substituted-5,6-di-hydroxyindole is N-isopropyl-5,6-dihydroxyindole.

13. The method of any preceding claim wherein the N-substituted-5,6-di-hydroxyindole is N-(2,4-dinitrophenyl)-5,6-dihydroxyindole.

14. The method of any preceding claim wherein the N-substituted-5,6-di-hydroxyindole is N-(4-nitrophenyl)-5,6-dihydroxyindole.

15. The method of any preceding claim wherein the N-substituted-5,6-di-hydroxyindole is N-(4-aminophenyl)-5,6-dihydroxyindole.

## Patentansprüche

1. Verfahren zum Färben von Haar, in dem mit dem Haar eine Lösung eines wasserlöslichen Metallions und danach eine Lösung eines N-substituierten 5,6-Dihydroxyindols oder in der umgekehrten Reihenfolge in Berührung gebracht wird, wobei der Substituent an dem Stickstoffatom aus der Gruppe ausgewählt ist, die aus Alkyl, Alkoxy, Hydroxyalkyl, Aminoalkyl, Aminoaryl und Nitroaryl besteht, wobei die Arylgruppe 1 bis 3 Substituenten tragen kann.

2. Verfahren nach Anspruch 1, in der die Lösung des N-substituierten 4,5-Dihydroxyindols eine Lösung von 0,1 bis 2,0 Gew.-% und die Lösung des wasserlöslichen Metallions eine Lösung von 0,001 bis 2,0 Gew.-% ist.

3. Verfahren nach einem der vorhergehenden Ansprüche, in dem die wasserlöslichen Salze aus der Gruppe ausgewählt sind, die aus den Salzen von Cr, Ag, Au, Pb, Pd, Co, Cu, Ni, Bi, Mn, Fe, Ti, Sn oder Zn besteht und in Lösung mit einer Konzentration von 0,001 bis 2,0 Gew.% und mit einem pH-Wert von 3 bis 11 aufgetragen werden, bevor das Haar mit der Lösung des N-substituierten 5,6 -Dihydroxyindols behandelt wird.

4. Verfahren nach einem der vorhergehenden Ansprüche, in dem die Lösung das N-substituierten 5,6-Dihydroxyindols bei einem pH-Wert von 5 bis 12 aufgetragen wird.

5. Verfuhren nach Anspruch 3, in dem das zu färbende Haar folgenden Schritten unterworfen wird: (1) Behandlung mit einer Zusammensetzung, die wasserlösliche Salze von Cr, Ag, Au, Pb, Pd, Co, Cu, Ni, Bi, Mn, Fe, Ti, Sn oder Zn enthält, die in Lösung mit Konzentrationen von 0,001 bis 2 Gew.-% und mit einem pH-Wert von 3 bis 11 aufgetragen werden; (2) darauffolgende Behandlung mit der Lösung des N-substituierten 5,6-Dihydroxyindols mit einem pH-Wert von 3 bis 12 mit oder ohne Spülen der zuerst aufgetragenen Lösung.

6. Verfahren nach Anspruch 5, in dem die Schritte (1) und (2) in umgekehrter Reihenfolge durchgeführt werden.

7. Verfahren nach einem der vorhergehenden Ansprüche mit der darauffolgenden 5- bis 30-minütigen Behandlung das Haares mit einer Lösung eines Bleichmittels mit einer Konzentration von 1 bis 6% und einem pH-Wert von 6 bis 11, damit die Färbung des Haares vermindert oder entfernt wird.

8. Verfahren nach Anspruch 7, in dem das Bleichmittel $H_2O_2$ ist.

9. Verfahren nach Anspruch 7, in dem das Bleichmittal ein Salz des $SO_2$ ist, z.B. Natrium- oder Kaliumbisulfat oder eine Sulfhydrylverbindung, z.B. Thioglykolsäure oder ihre Kalium-, Natrium- oder Ammoniumsalze.

10. Verfahren nach einem der vorhergehenden Ansprüche in dem in dem N-substituierten 5,6-Dihydroxyindol der Substituent Methyl, Isopropyl oder ein Aminophenyl ist oder ein Nitrophenyl, in dem jeder

Benzolring ebenfalls 1 bis 3 Substituenten haben kann.

**11.** Verfahren nach einem der vorhergehenden Ansprüche, in dem das N-substituierte 5,6-Dihydroyindol N-Methyl-5,6-dihydroxyindol ist.

**12.** Verfahren nach einem der vorhergehenden Ansprüche, in dem das N-substituierte 5,6-Dihydroxyindol N-Isopropyl-5,6-dihydroxyindol ist.

**13.** Verfahren nach einem der vorhergehenden Ansprüche, in dem das N-substituierte 5,6-Dihydroxyindol N-(2,4-dinitrophenyl)-5,6-dihydroxyindol ist.

**14.** Verfahren nach einem der vorhergehenden Ansprüche, in dem des N-substituierte 5,6-Dihydroxyindol N-(4-Nitrophenyl)-5,6-dihydroxyindol ist.

**15.** Verfahren nach einem der vorhergehenden Ansprüche, in dem des N-substituierte 5,6-Dihydroxyindol N-(4-aminophenyl)-5,6-dihydroxyindol ist.

**Revendications**

**1.** Une méthode de coloration de la chevelure, qui comprend la mise en contact de la chevelure avec une solution d'un ion métallique soluble dans l'eau, suivie du contact de la chevelure avec une solution de 5,6-dihydroxyindole N-substituée, ou dans l'ordre inverse, dans laquelle le substituant sur l'atome d'azote est sélectionné parmi les groupes consistant en alcoxyalkyle, hydroxyalkyle, aminoalkyle, aminoaryle et nitroaryle, les groupes aryles pouvant comporter jusqu'à 1 à 3 substituants.

**2.** La méthode de la revendication 1, dans laquelle une solution contient de 0,1 à 2% en poids de 5,6-dihydroxyindole N-substituée et dans laquelle ladite solution d'ion métallique soluble dans l'eau contient de 0,001 à 2,0% en poids.

**3.** La méthode selon l'une quelconque des revendications précédentes, dans laquelle les sels solubles dans l'eau sont sélectionnés dans le groupe composé des sels de Cr, Ag, Au, Pb, Pd, Co, Cu, Ni, Bi, Mn, Fe, Ti, Sn ou Zn, et sont appliqués préalablement au traitement de la chevelure avec ladite solution de 5,6-dihydroxyindole N-substituée, sous forme de solution dont les concentrations vont de 0,001% à 2,0% en poids, à un pH compris entre de pH 3 et pH 11.

**4.** La méthode selon l'une quelconque des revendications précédentes, dans laquelle la solution de 5,6-dihydroxyindole N-substituée est appliquée à un pH compris entre 3 et 12.

**5.** La méthode selon la revendication 3, dans laquelle les cheveux devant être teintés sont soumis aux étapes comprenant:
(1) le traitement à l'aide d'une composition comprenant des sels solubles dans l'eau de Cr, Ag, Au, Pb, Pd, Co, Cu, Ni, Bi, Mn, Fe, Ti, Sn ou Zn, appliqués sous la forme d'une solution dont les concentrations vont de 0,001% à 2,0% en poids, et à un pH compris entre pH 3 et pH 11;
(2) un traitement ultérieur avec ladite solution de 5,6-dihydroxyindole N-substituée à un pH allant de pH 3 à pH 12, avec ou sans rinçage de la première solution appliquée.

**6.** La méthode selon la revendication 5, dans laquelle l'ordre des étapes (1) et (2) est inversé.

**7.** La méthode selon l'une quelconque des revendications précédentes, suivie par un traitement ultérieur de la chevelure avec une solution d'un agent décolorant, à une concentration de 1% à 6% et à un pH de 6 à 11, pendant une période de 5 à 30 minutes pour obtenir une décoloration ou un éclaircissement de la coloration de la chevelure.

**8.** La méthode selon la revendication 7, dans laquelle l'agent décolorant est $H_2O_2$.

**9.** La méthode selon la revendication 7, dans laquelle l'agent décolorant est un sel de $SO_2$ tel que bisulfate de sodium ou de potassium ou un dérivé sulfhydrilique tel que l'acide thioglycolique ou ses sels d'ammonium, de sodium ou de potassium.

**10.** La méthode selon l'une quelconque des revendications précédentes, dans laquelle dans la 5,6-dihydroxyindole N-substituée le substituant est le méthyle, l'isopropyle, l'aminophényle ou le nitrophényle, dans lesquels chaque cycle benzène peut également avoir 1 à 3 substituants.

**11.** La méthode selon l'une quelconque des revendications précédentes, dans laquelle la 5,6-dihydroxyindole N-substituée est la N-méthyl-5,6-dihydroxyindole.

**12.** La méthode selon l'une quelconque des revendications précédentes, dans laquelle la 5,6-dihydroxyindole N-substituée est la N-isopropyl-5,6-dihydroxyindole.

**13.** La méthode selon l'une quelconque des revendications précédentes, dans laquelle la 5,6-dihydroxyindole N-substituée est la N-(2,4-dinitrophényl)-5,6-dihydroxyindole.

**14.** La méthode selon l'une quelconque des revendications précédentes, dans laquelle la 5,6-dihydroxyindole N-substituée est la N-(4-nitrophényl)-5,6-dihydroxyindole.

**15.** La méthode selon l'une quelconque des revendications précédentes, dans laquelle la 5,6-dihydroxyindole N-substituée est la N-(4-aminophényl)-5,6-dihydroxyindole.